# EUROPEAN PATENT SPECIFICATION

(11) **EP 1 756 140 B1**
(45) Date of publication and mention of the grant of the patent: **09.03.2011**
(21) Application number: 05744278.2
(22) Date of filing: 20.05.2005
(51) Int. Cl.: C07J 17/00, A61K 31/70, A61P 3/04

(54) **SELECTIVE SEPARATION OR EXTRACTION OF STEROIDAL GLYCOSIDES BY SUPERCRITICAL FLUID EXTRACTION USING CARBON DIOXIDE**
SELEKTIVE ABTRENNUNG ODER EXTRAKTION VON STEROIDALEN GLYCOSIDEN DURCH EXTRAKTION MIT SUPERKRITISCHER FLÜSSIGKEIT UNTER EINSATZ VON KOHLENDIOXID
SEPARATION OU EXTRACTION SELECTIVE DE GLYCOSIDES STEROIDIQUES PAR EXTRACTION DE FLUIDE SUPER CRITIQUE UTILISANT DU DIOXYDE DE CARBONE

(30) Priority: 25.05.2004 GB 0411703
(43) Date of publication of application: 28.02.2007
(73) Proprietor: Unilever N.V., 3013 AL Rotterdam (NL)
(72) Inventor: GUNNING, Philip James, c/o Phytopharm plc, Godmanchester, Cambridgeshire PE29 2HY (GB); ROSE, Paul Martin, c/oBotanix Limited, Paddock Wood, Kent TN12 6DQ (GB); MARRIOT, Raymond,John, c/oBotanix Limited, Paddock Wood, Kent TN12 6DQ (GB)
(74) Representative: Brown, David Leslie
(86) International application number: PCT/GB2005/001995
(87) International publication number: WO 2005/116049

(56) References cited:
- WO-A-98/46243
- WO-A-03/041727
- WO-A-2004/071399
- US-A1- 2003 152 648
- DINAN L ET AL: "Chromatographic procedures for the isolation of plant steroids" JOURNAL OF CHROMATOGRAPHY A, ELSEVIER SCIENCE, NL, vol. 935, no. 1-2, 23 November 2001 (2001-11-23), pages 105-123, XP004322067 ISSN: 0021-9673
- W. N. MOORE ET AL: "Solid Phase Trapping Mechanisms Involved in the Supercritical Fluid Extraction of Digitalis Glycosides with Modified Carbon Dioxide" ANALYTICAL CHEMISTRY., vol. 67, no. 13, 1 July 1995 (1995-07-01), pages 2030-2036, XP002302079 USAMERICAN CHEMICAL SOCIETY. COLUMBUS.
- MOORE W N ET AL: "Extraction and quantitation of digoxin and acetyldigoxin from the Digitalis lanata leaf via near-supercritical methanol-modified carbon dioxide" JOURNAL OF NATURAL PRODUCTS 1996 UNITED STATES, vol. 59, no. 7, 1996, pages 690-693, XP002302080 ISSN: 0163-3864

## Description

### Field of the Invention

The present invention relates to the selective separation or extraction of steroidal glycosides from materials containing more than one of these compounds and optionally other components. Most particularly, such materials may be plant matter in which the steroidal glycosides occur naturally.

### Background of the Invention

Extracts obtainable from plants of the Asclepiadaceae family, particularly the *Hoodia* genus (formerly the *Hoodia* and *Trichocaulon* genera), have been shown to contain the steroid glycoside **1** which is an appetite suppressing compound (PCT application WO 98/46243).

An extraction process is outlined in the prior application, involving treating plant material with a solvent to extract a fraction having appetite suppressant activity, separating the extraction solution from the rest of the plant material, removing the solvent from the extraction solution and recovering the extract. The solvents specifically mentioned to perform the extraction are one or more of methylene chloride (dichloromethane), water, methanol, hexane, ethyl acetate or mixtures thereof.

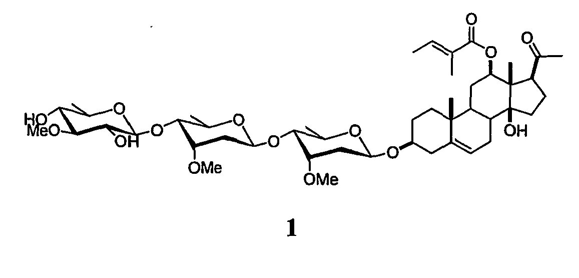

The prior art solvent extraction process tends, however, to result in a mixture of extracted compounds, not all of which have desirable properties. Typically, some may have an unpleasant taste.

The need for a subsequent fractionation or separation procedure to remove undesirable extracted compounds leads to increased manufacturing costs and complicated procedures, which it is preferable to avoid.

US Patent Application No. US-A-2003/0152648, the disclosure of which is incorporated herein by reference, describes extraction of pregnane glycosides, particularly stavarosides, from the plant *Orbea variegata,* a member of the Asclepiadaceae family. The extraction process involves initial solvent extraction from dried and ground plant material, using a suitable solvent. It is stated generally that "suitable solvents include water, dilute acids, organic solvents, critical, supercritical or near critical -fluid solvents, e.g. carbon dioxide, nitrous oxide, propane, ethane, ethylene and fluorohydrocarbons, and mixtures of any of these". The preferred solvent is stated to be an alcohol based solvent, the most preferred being ethanol. Ethanol extraction is the only technique shown in any working example. The solvent extraction technique is stated to be usable to extract stavarosides from the plant material, and there is no teaching or suggestion to use it to selectively separate desirable steroidal glycosides from undesirable steroidal glycosides.

Moore and Taylor (J. Nat. Prod 1996, 59, 690-693), the disclosure of which is incorporated herein by reference, describe the extraction of the cardiac glycosides, digoxin and acetyldigoxin, from *Digitalis lanata* using near supercritical methanol-modified carbon dioxide. The authors indicate that to effect efficient extraction the extraction solvent was methanol and carbon dioxide where the methanol was in the range of 10-25%, with the optimised conditions employing 20% methanol modified CO₂

We have now found, surprisingly, that, by using a particular extraction technique described below, specifically the use of liquid or supercritical carbon dioxide, a relatively efficient selective extraction of desirable steroidal glycosides from Asclepiadaceae plant material can be achieved, which involves the selective separation of desirable steroidal glycosides from undesirable steroidal glycosides, reducing or eliminating the need for subsequent processing to remove undesirable components.

### Brief Description of the Invention

The present invention provides a method for selectively separating a steroidal glycoside of formula 1 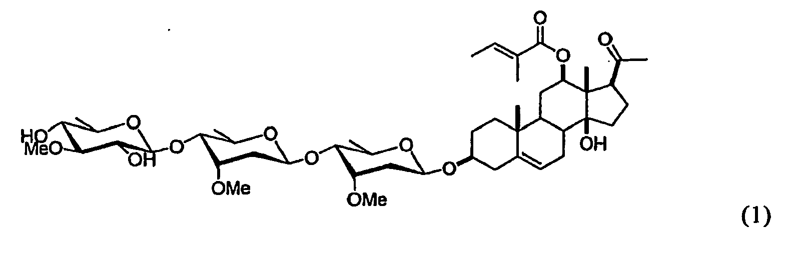 from one or more steroidal glycosides selected from compounds 3 to 13 as defined below or other compounds, present in plant material of the Asclepiadaceae family containing the same, the method comprising contacting the plant material or material derived therefrom, hereinafter referred to as "the material", with liquid or supercritical carbon dioxide under conditions whereby the steroidal glycoside of formula 1 dissolves in the liquid or supercritical carbon dioxide in preference to the one or more of compounds 3 to 13 or other compounds, and subsequently recovering the steroidal glycoside of formula I from the carbon dioxide solution extracted substantially free of any unpleasant tasting component of the material and substantially free of one or more of compounds 3 to 13:

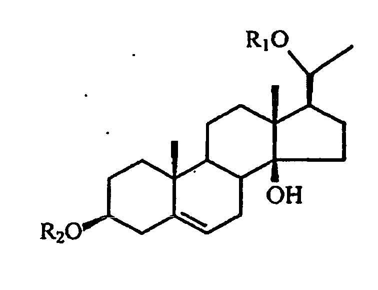

| **Compound** | **R₁** | **R₂** |
|---|---|---|
| **3** | glu-glu-glu- | tig-the-ole- |
| **4** | glu-glu- | tig-mda-cym- |
| **5** | glu-glu-glu- | tig-cym-cym- |
| **6** | glu-glu-glu- | tig-ole-cym-cym-cym- |
| **7** | glu-glu-glu- | tig-ole-cym- |
| **8** | glu-glu-glu- | ang-mda-cym- |
| **9** | glu-glu-glu- | tig-the-cym- |
| **10** | glu-glu-glu- | tig- |
| **11** | glu-glu-glu- | tig-cym- |
| **12** | glu-glu-glu- | tig-the-ole-cym- |
| **13** | glu-glu-glu- | tig-the-cym-cym- |

| | | |
|---|---|---|
| ole = oleandrose, cym = cymarose, glu = glucose, mda = 3-O-methyl-6-deoxyallose, tig = tigloyl, ang = angeloyl, the = thevetose. | | |

In the following description of the invention, the steroidal glycoside of formula 1 will be referred to as a "desirable steroidal glycoside" and the steroidal glycosides which are separated from it will be referred to as the "undesirable steroidal glycosides".

The use of liquid or supercritical carbon dioxide as an extraction solvent is known, and apparatus for performing the technique are readily available. For a general review of the technology, please refer to Kaiser et al, Pharmazie 56, 907-926 (2001) and Lang et al, Talanta 52, 771-782 (2001)

The conditions of the extraction which may be varied to control the selectivity are primarily the temperature and the pressure of the sealed extraction apparatus. However, the prior art has generally restricted the use of the technique to extraction of active compounds from inactive cell-wall and other material and the use of liquid or supercritical carbon dioxide as a means for selectively separating desirable steroidal glycosides from relatively chemically similar undesirable steroidal glycosides in plant material of the Asclepiadaceae family was not previously known or suggested.

After an initial extraction from the material, further extraction cycles can be performed on the remaining material if desired, using fresh carbon dioxide, in order to maximise the yield of extracted compounds.

An initial extraction can be performed using liquid carbon dioxide, and a subsequent extraction performed on the remaining material using supercritical carbon dioxide. Alternatively, both an initial and a subsequent extraction can use liquid carbon dioxide. Still further, both an initial and a subsequent extraction can use supercritical carbon dioxide. The conditions of these sequential extractions can be chosen to preferentially remove particular components from the material.

The method of the present invention may, if desired, be performed using a flow of carbon dioxide with downstream recovery of the desirable steroidal glycosides.

The method of the present invention can be used with other extraction procedures for removing other, e.g. non-steroidal, components from the material to be treated. Thus, for example, an initial extraction using liquid carbon dioxide can be performed under conditions selected to preferentially remove undesirable lipophilic compounds such as fatty acids from the material. A subsequent extraction performed on the remaining material according to the present invention can thereby result in an improved yield of the desirable steroidal glycoside.

An extract produced by the method of the invention can, if desired, be subjected to one or more subsequent extractions, e.g. one or more subsequent solvent extractions using one or more organic solvent to remove any residual unwanted compounds, for example non-steroidal glycosides. The choice of organic solvent(s) will be well within the capacity of a person of ordinary skill in this art, considering the materials being treated. The said one or more subsequent solvent extractions may suitably be performed using organic solvents selected from acetone, ethyl acetate, heptane (e.g. n-heptane), ethanol, hexane (e.g. n-hexane) and any mixture or combination thereof, and at any convenient temperature.

The material on which the method of the present invention may be performed may be plant material or material derived therefrom. The term "plant material" in this context includes all forms of plant material, including freshly cut plant material, dried or preserved plant material, which may optionally be comminuted, e.g. powdered or crushed. The term "material derived therefrom" in this context includes extracts and decoctions obtained from the plant material. Such extracts may, for example, be obtainable by a non-selective extraction method, such as the prior art solvent extraction method.

The steroidal glycoside of formula 1 has desirable bioactivity, for example having bioactivity to suppress appetite (particularly through actions at centres in the brain controlling of feelings of hunger and fullness), to treat excessive body weight (particularly above-average body weight for an individual's age and height), to treat obesity, to reduce total calorific intake of an individual (particularly, total calorific intake over an extended period of at least about two weeks in a lifestyle or environment where calorific foodstuffs and beverages are available substantially *ad libitum),* or any combination thereof.

More particularly, the plant may be a plant of the *Hoodia* genus. The plant of the *Hoodia* genus may suitably be selected from *Hoodia gordonii, Hoodia currorii* subsp. *currorrii* and *Hoodia currorii* subsp. *lugardii.* The plant of the *Hoodia* genus is preferably *Hoodia gordonii.*

The undesirable steroidal glycosides or other compounds, from which the desirable steroidal glycoside is to be separated according to the method of the present invention, may, for example, comprise an unpleasant tasting component.

A multi-step extraction process may use conditions of differing temperature and/or pressure at different stages. Most particularly, conditions of differing pressure may be used. The present invention therefore includes within its scope the use of a multi-pressure extraction of dried plants of the *Hoodia* genus wherein the plant material is initially extracted with carbon dioxide under liquid conditions to extract unwanted lipophilic material such as fatty acids, and then subsequently extracted with carbon dioxide under supercritical conditions to afford an extract containing elevated levels of compound 1, whilst leaving unwanted steroidal glycosides or other compounds with the plant matrix.

If desired, one or more aqueous or organic co-solvent may be used in the method of the present invention, in conjunction with the carbon dioxide solvent and under the same conditions of temperature and pressure as applied to the carbon dioxide. Any such co-solvent that is present will be used in generally small amounts relative to the carbon dioxide, for example less than about 10% by weight (e.g. about 5% by weight) relative to the amount of carbon dioxide used. The co-solvent may suitably be selected from water, methanol, ethanol, hexane (e.g. n-hexane), heptane (e.g. n-heptane) and any mixture or combination thereof.

The present invention thus enables steroidal glycosides to be provided, which have been separated from plant material by means of the method of the present invention.

### Detailed Description of the Invention

The taxonomy of plants of the Asclepiadaceae family has been revised in recent years. For example, in 1992 Bruyns reclassified a number of plants to the genus *Hoodia,* which were previously in the genus *Trichocaulon* (Bruyns; Bot. Jahrb. Syst. 115 (2) 145-270 (1993)). One such reclassification related to *Hoodia pilifera,* previously *Trichocaulon piliferum.* The genus *Trichocaulon* now does not exist.

Work undertaken on identifying molecules present in plants belonging to the genus *Hoodia* has shown that compound 1 is present in addition to a set of steroid glycosides covered by the generic structure 2.

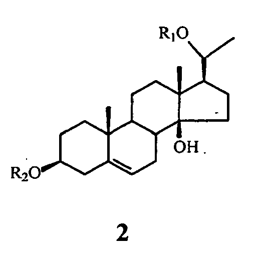

For example, from *Hoodia gordonii* eleven compounds have been isolated and characterised (Compounds **3-13**).

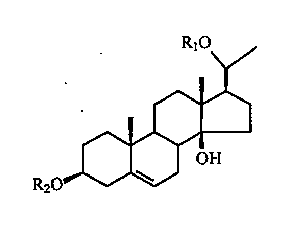

| **Compound** | **R₁** | **R₂** |
|---|---|---|
| **3** | glu-glu-glu- | tig-the-ole- |
| **4** | glu-glu- | tig-mda-cym- |
| **5** | glu-glu-glu- | tig-cym-cym- |
| **6** | glu-glu-glu- | tig-ole-cym-cym-cym- |
| **7** | glu-glu-glu- | tig-ole-cym- |
| **8** | glu-glu-glu- | ang-mda-cym- |
| **9** | glu-glu-glu- | tig-the-cym- |
| **10** | glu-glu-glu- | tig- |
| **11** | glu-glu-glu- | tig-cym- |
| **12** | glu-glu-glu- | tig-the-ole-cym- |
| **13** | glu-glu-glu- | tig-the-cym-cym- |

| | | |
|---|---|---|
| ole = oleandrose, cym = cymarose, glu = glucose, mda = 3-O-methyl-6-deoxyallose, tig = tigloyl, ang = angeloyl, the = thevetose. | | |

Methanol extraction of dried *Hoodia gordonii* plant material affords extracts containing compound **1** and compounds **3-13**. The use of other solvents such as dichloromethane or ethyl acetate similarly produces extracts containing all the steroidal glycosides.

Surprisingly, it has been found that liquid or supercritical carbon dioxide extraction (for example at pressures of about 300 to about 500 bar and at a temperature of about 55 to about 80°C) affords selective extraction of compound **1** whilst retaining compounds **3-13** in the plant matrix. The pressure and temperature can be varied outside these limits.

Certain members of the genus *Hoodia* are known to contain bitter components making the palatability of the plant or extracts thereof poor. Indeed, the native names for *Hoodia gordonii* and *Hoodia currorii* subsp. *lugardii* is 'bitterghaap' which may be translated as bitter food. The use of *Hoodia gordonii* as an appetite suppressing agent could be hindered by this bitterness. Surprisingly, it has been found that extraction of plants of the genus *Hoodia* using carbon dioxide extraction affords an extract rich in compound 1 but lacking any unpleasant taste.

A second aspect of the invention is the use of a multi-pressure extraction of dried plants of the *Hoodia* genus wherein the plant material is initially extracted with carbon dioxide under liquid conditions to extract unwanted lipophilic material such as fatty acids, and then subsequently extracted with carbon dioxide under supercritical conditions to afford an extract containing elevated levels of compound 1, whilst leaving unwanted steroid glycosides, for example compounds **3-13** with the plant matrix.

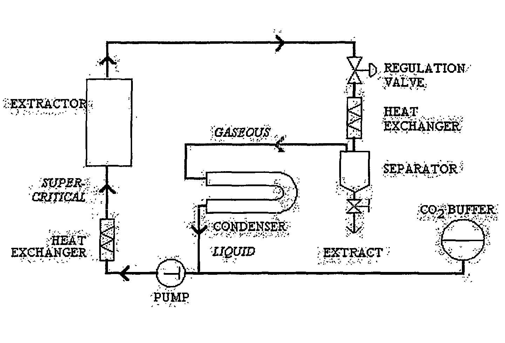

### Brief Description of the Drawing

The accompanying drawing shows, purely by way of illustration and without limitation, a suitable carbon dioxide extraction system for use in performing the present invention.

### Detailed Description of the Drawing

A suitable extraction unit (see drawing) consists of extractor and separator vessels as well as of different heat exchangers, pumps, regulation valves and devices. The extractor vessel is filled with the raw material which is to be treated. The required pressure is achieved through a solvent which flows in from a tank. For increasing the pressure, a pump is necessary, which also takes over the transport of the solvent after the required extraction pressure has been achieved. The pressure is maintained by an overflow valve which opens when the required pressure is exceeded and transports the enriched solvent to the separator step. Usually, this separation step is connected with the storage tank of the solvent. The pressure in the separator step and in the storage tank will consequently be the same (with the exception of flow losses) and will correspond to the saturation (evaporation) pressure of the solvent at the respective temperature.

### Examples

The following Examples illustrate, without limitation, the selective extraction of steroidal glycosides from plants of the Asclepiadaceae family using carbon dioxide as the solvent.

### Example 1

Dried *Hoodia gordonii* plant material was milled to a fine powder (< 1 mm). 700 g of milled material was packed into a single extractor column and extracted with CO₂ at 300 bar and 55°C. Table 1 indicates the quantity of extract obtained during the extraction, as well as the results of the HPLC analysis of the extracts.

**Table 1 Yield and composition during the extraction of dried Hoodia gordonii plant material with carbon dioxide at 300 bar and 55°C**

| Extraction Time (hours) | Yield (g) | Total Yield (g) | Total Yield % | Content of compound 1 (%) |
|---|---|---|---|---|
| 0.5 | 18.53 | 18.53 | 2.65 | 0.6 |
| 1.0 | 7.70 | 26.23 | 3.75 | 1.3 |
| 2.0 | 6.88 | 33.11 | 4.73 | 1.1 |
| 3.0 | 5.28 | 38.39 | 5.48 | 1.4 |
| 4.0 | 1.21 | 39.60 | 5.66 | 3.0 |
| 5.0 | 2.26 | 41.86 | 5.98 | 2.3 |
| 6.0 | 0.53 | 42.39 | 6.06 | Not determined |

### Example 2

Dried *Hoodia gordonii* plant material was milled to a fine powder. Particle size analysis of this material showed that 85% passed a 600 µm mesh. 700 g of milled material was packed into a single extractor column and extracted sequentially with CO₂ at increasing pressure and temperature. Table 2 indicates the quantity of extract obtained at the four experimental conditions, as well as the results of the HPLC and GC analysis of the extracts.

**Table 2: Yield and composition under increasing pressure and temperature conditions of carbon dioxide**

| | Extracted weight (g) | % of nine identified steroid glycosides | % of compound 1 | % triglycerides | % hydrocarbons |
|---|---|---|---|---|---|
| 60 bar 10°C | 5.000 | 10.9 | 1.2 | 39.3 | 1.4 |
| 100 bar 25°C-35°C | 13.510 | 4.3 | 0.7 | 17.2 | 1.3 |
| 200 bar 35°C | 1.600 | 36.5 | 8.8 | 11.0 | 14.8 |
| 300 bar 55°C | 13.290 | 19.2 | 4.6 | 17.9 | 7.9 |

### Example 3

A series of extractions were performed using standardised conditions of extraction of milled *Hoodia gordonii* plant material with carbon dioxide at 100 bar at 25°C-35°C (LCO₂ for 7 hours, followed by extraction at 300 bar at 55°C (SCO₂) for 7 hours. The two extracts obtained were analysed by HPLC to determine that levels of steroid glycosides. Table 3 shows the quantity of input material per batch and the percentage of compound 1 in the two extracts obtained for each of the extraction experiments.

The input batches of dried *Hoodia gordonii* contain 0.06-0.2 % of compound **1**. Extraction with liquid carbon dioxide (LCO2) affords an extract in a yield of 0.71-1.94% that contains 0.6-2.7% of compound **1**. Compounds **3-13** were not detected in the product. Sequential extraction with supercritical carbon dioxide (SCO2) affords a yellow-green powder which has no bitter taste in a yield of 0.67-2.34 % that contains 2.2-5.7% of compound 1. Again compounds **3-13** were not detected in the product.

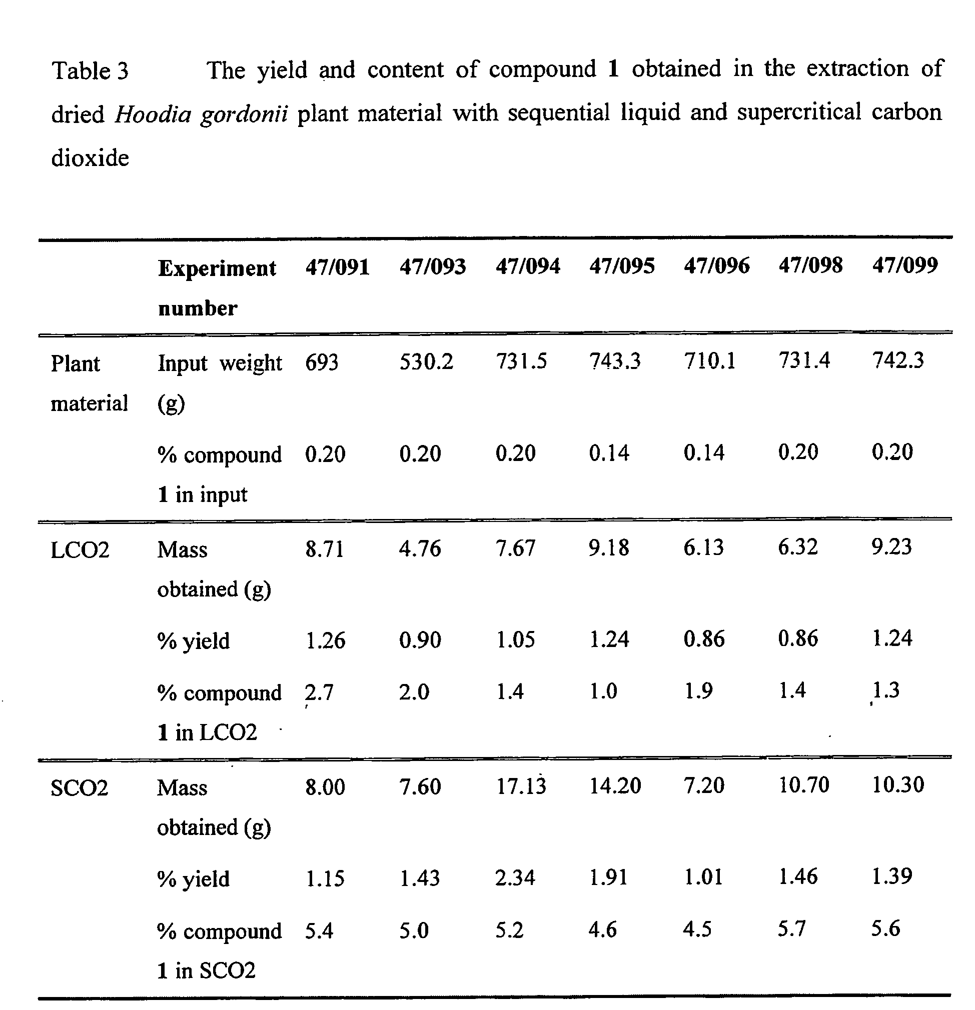

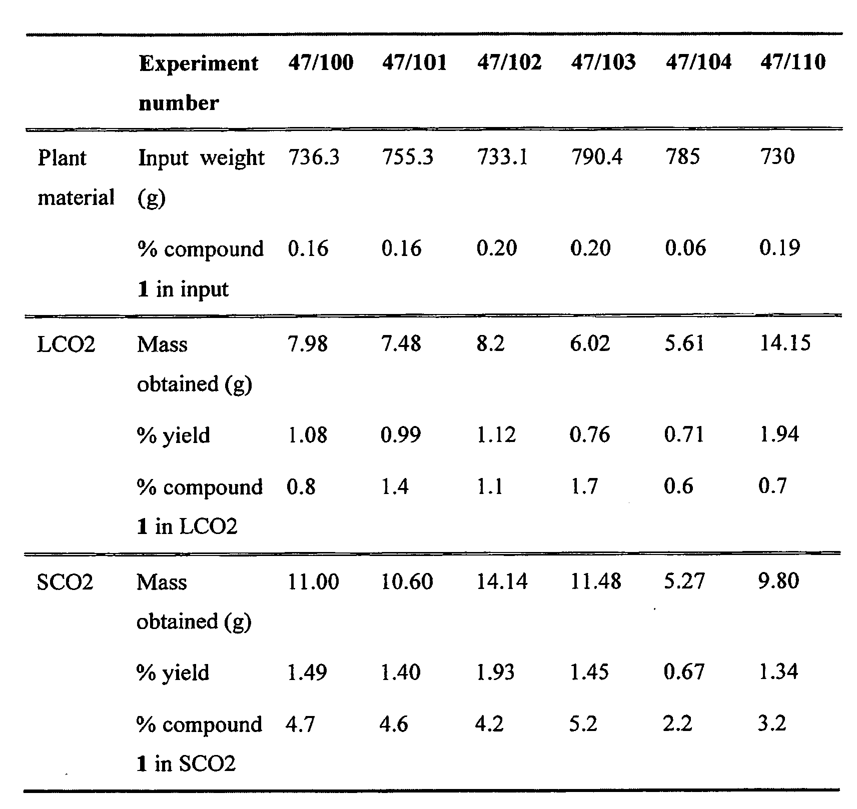

### Example 4

A series of extractions were performed using standardised conditions of extraction of milled *Hoodia gordonii* plant material with carbon dioxide at a pressure of 100 bar. Material was milled using a coffee grinder and sieved to between 106 and 600 µm. The ground material was dried at 70°C for a minimum of 16 hours to remove any moisture. The series of experiments used plant material of 650 - 810 g. The extraction pressure was maintained at 100 bar with a CO₂ flow rate of 5 kg/h for an extraction period of 7 hours. The product was removed from the separator by washing with methanol. The methanol washings were evaporated to dryness to obtain an accurate value for the extraction mass yield. The extraction efficiency of compound 1 was obtained by HPLC analysis of the methanol washings and compared to the amount of compound **1** in the input material. Table 4 shows the quantity of input material per batch and the percentage of compound **1** in the extracts obtained for each of the extraction experiments where the extraction temperature was varied from 5 to 75°C. Compounds **3-13** were not detected in the product.

**Table 4 The extraction efficiency for compound 1 obtained in the extraction of dried Hoodia gordonii plant material at a variety of temperatures with liquid or supercritical carbon dioxide at a fixed pressure of 100 bar**

| Charge weight (g) | Temperature (°C) | Mass of extract (g) | Compound 1 in extract (%) | Extraction efficiency for compound 1 (%) |
|---|---|---|---|---|
| 807.6 | 5 | 9.69 | 2.75 | 18.33 |
| 717.4 | 15 | 6.60 | 2.71 | 13.85 |
| 707.4 | 25 | 8.77 | 1.53 | 10.54 |
| 712.5 | 35 | 8.69 | 0.46 | 3.12 |
| 725.7 | 40 | 11.97 | 0.15 | 1.65 |
| 723.7 | 45 | 3.62 | 0 | 0.00 |
| 657.6 | 55 | 0.59 | 0.14 | 0.07 |

### Example 5

A series of extractions were performed on milled *Hoodia gordonii* plant material with carbon dioxide at a pressure of 300 bar. Material was milled using a hammermill passing through a 2 mm mesh sieve. The extraction pressure was maintained at 300 bar with a CO₂ feed ratio of 100 kg CO₂/kg input for an extraction period of ca 7 hours. The extraction efficiency of compound **1** was obtained by HPLC analysis of the spent plant material and compared to the amount of compound **1** in the input material. Table 5 shows the quantity of input material per batch and the extraction efficiency of compound **1** for each of the extraction experiments where the extraction temperature was varied from 0 to 90°C. Compounds **3-13** were not detected in the product.

**Table 5 The extraction efficiency for compound 1 obtained in the extraction of dried Hoodia gordonii plant material at a variety of temperatures with liquid or supercritical carbon dioxide at a fixed pressure of 300 bar**

| Charge weight (g) | Temperature (°C) | Extraction efficiency for compound 1 (%) |
|---|---|---|
| 87.54 | 0 | 27 |
| 88.55 | 20 | 31 |
| 87.52 | 40 | 48 |
| 85.92 | 60 | 53 |
| 85.02 | 80 | 59 |
| 84.75 | 90 | 60 |

### Example 6

A series of extractions were performed on milled *Hoodia gordonii* plant material with supercritical carbon dioxide exploring the effects of high temperatures and pressures, the input particle size, and the presence of co-solvents. Material was milled using a hammermill passing through either a 0.7, 1 or 2 mm mesh sieve. The extraction pressure was maintained set at either 300 or 500 bar with a CO₂ feed ratio of 100 kg CO₂/kg input for an extraction period of ca 7 hours. The extraction efficiency of compound **1** was obtained by HPLC analysis of the spent plant material and compared to the amount of compound **1** in the input material. Table 6 shows the quantity of input material per batch and the extraction efficiency of compound **1** for each of the extraction experiments. Compounds **3-13** were not detected in the product.

**Table 6 The extraction efficiency for compound 1 obtained in the extraction of dried Hoodia gordonii plant material at a variety of temperatures, pressures, input particle size with supercritical carbon dioxide in the presence or absence of co-solvents**

| Charge weight (g) | Pre-treatment sieve size (mm) | Temperature (°C) | Pressure (bar) | Presence of co-solvent | Extraction efficiency for compound 1 (%) |
|---|---|---|---|---|---|
| 85.02 | 2 | 80 | 300 | None | 59 |
| 84.92 | 1 | 80 | 300 | None | 63 |
| 84.41 | 0.7 | 80 | 300 | None | 70 |
| 42.08 | 2 | 80 | 300 | None | 62 |
| 85.76 | 1 | 80 | 300 | 5% EtOH | 80 |
| 84.29 | 1 | 80 | 500 | None | 81 |
| 83.23 | 0.7 | 80 | 500 | None | 83 |
| 82.88 | 0.7 | 80 | 500 | 5%EtOH | 88 |
| 83.86 | 0.7 | 80 | 500 | None | 78 |
| 84.08 | 0.7 | 80 | 500 | 5% EtOH | 84 |
| 77.14 | 0.7 | 80 | 500 | None | 84 |
| 61.76 | 0.7 | 80 | 500 | 5% EtOH | 84 |
| 59.34 | 0.7 | 100 | 500 | None | 84 |
| 78.33 | 0.7 | 80 | 500 | 5% EtOH | 86 |
| 65.45 | 0.7 | 80 | 500 | 5% EtOH | 80 |

The foregoing broadly describes the present invention, without limitation. Variations and modifications as will be readily apparent to those of ordinary skill in the art are intended to be included in the scope of this application and subsequent patent(s).

## Claims

1. A method for selectively separating a steroidal glycoside of formula 1 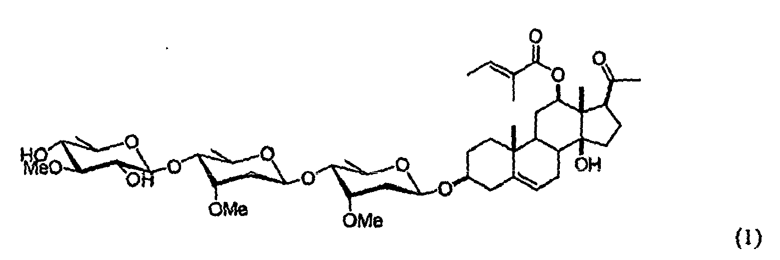 from one or more steroidal glycosides selected from compounds 3 to 13 as defined below or other compounds, present in plant material of the Asclepiadaceae family containing the same, the method comprising contacting the plant material or material derived therefrom, hereinafter referred to as "the material", with liquid or supercritical carbon dioxide under conditions whereby the steroidal glycoside of formula I dissolves in the liquid or supercritical carbon dioxide in preference to the one or more of compounds 3 to 13 or other compounds, and
subsequently recovering the steroidal glycoside of formula I from the carbon dioxide solution extracted substantially free of any unpleasant tasting component of the material and substantially free of one or more of compounds 3 to 13: 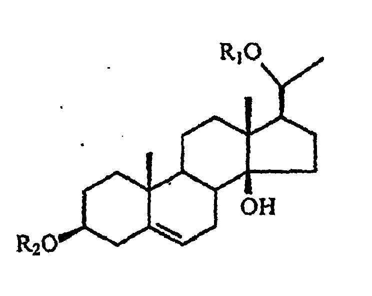
| **Compound** | **R₁** | **R₂** |
|---|---|---|
| **3** | glu-glu-glu- | tig-the-ole- |
| **4** | glu-glu- | tig-mda-cym- |
| **5** | glu-glu-glu- | tig-cym-cym- |
| **6** | glu-glu-glu- | tig-ole-cym-cym-cym- |
| **7** | glu-glu-glu- | tig-ole-cym- |
| **8** | glu-glu-glu- | ang-mda-cym- |
| **9** | glu-glu-glu- | tig-the-cym- |
| **10** | glu-glu-glu- | tig- |
| **11** | glu-glu-glu- | tig-cym- |
| **12** | glu-glu-glu- | tig-the-ole-cym- |
| **13** | glu-glu-glu- | tig-the-cym-cym- |
| | | |
|---|---|---|
| ole = oleandrose, cym = cymarose, glu = glucose, mda = 3-O-methyl-6-deoxyallose, tig = tigloyl, ang = angeloyl, the = thevetose. | | |

2. A method according to claim 1, wherein predominantly fatty acids and other lipid material are extracted in a first extraction using liquid carbon dioxide at a first pressure, followed by extraction of steroidal glycoside of formula 1 using supercritical carbon dioxide at a second pressure whilst leaving steroidal glycosides selected from compounds 3 to 13 or other compounds with the plant matrix.

3. A method according to claim 1 or claim 2, wherein the carbon dioxide is used in conjunction with a relatively small amount of one or more aqueous or organic co-solvent, wherein the co-solvent is used in an amount less than about 10% by weight of the carbon dioxide.

4. A method according to claim 3, wherein the co-solvent is selected from water, methanol, ethanol, hexane, heptane and any mixture or combination thereof.

5. A method according to any one of the preceding claims, wherein the plant is a plant of the *Hoodia* genus.

6. A method according to claim 5, wherein the plant is selected from *Hoodia gordonii, Hoodia currorii* subsp. *currorrii* and *Hoodia currorii* subsp. *lugardii.*

7. A method according to claim 6, wherein the plant is *Hoodia gordonii.*

8. A method according to any one of the preceding claims, wherein an extract initially produced is subjected to one or more subsequent solvent extractions using one or more organic solvent to remove any residual unwanted compounds.

9. A method according to claim 8, wherein the one or more organic solvent is selected from acetone, ethyl acetate, heptane, ethanol, hexane and any mixture or combination thereof.

10. A method according to claim 8, wherein the one or more organic solvent is selected from acetone, heptane and any mixture or combination thereof.

## Patentansprüche

1. Verfahren zur selektiven Abtrennung eines steroidalen Glycosids der Formel 1 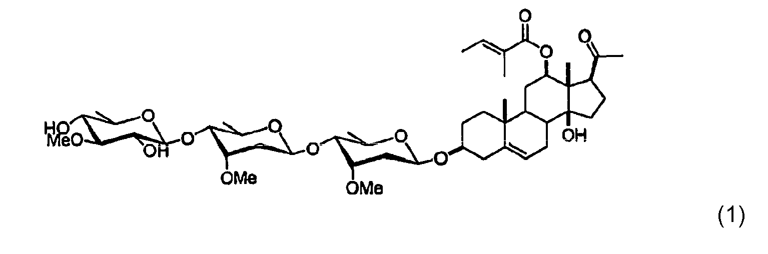 aus einem oder mehreren steroidalen Glycosiden, ausgewählt aus den unten definierten Verbindungen 3 bis 13, oder aus anderen Verbindungen, anwesend in Pflanzenmaterial der Asclepiadaceae-Familie, das gleiches enthält, das Verfahren umfassend Zusammenbringen des Pflanzenmaterials oder davon abgeleiteten Materials, ab jetzt bezeichnet als "das Material", mit flüssigem oder superkritischem Kohlendioxid unter Bedingungen, bei denen das steroidale Glycosid der Formel 1 sich in dem flüssigen oder superkritischen Kohlendioxid bevorzugt gegenüber der einen oder mehreren Verbindungen 3 bis 13 oder anderen Verbindungen löst, und anschließend Wiedergewinnen des steroidalen Glycosids der Formel 1 aus der Kohlendioxidlösung, extrahiert im Wesentlichen frei von unangenehm schmeckenden Bestandteilen des Materials, und im Wesentlichen frei von einer oder mehreren der Verbindungen 3 bis 13: 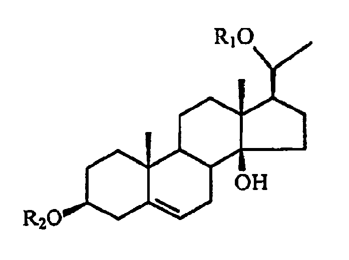
| **Verbindung** | **R₁** | **R₂** |
|---|---|---|
| **3** | glu-glu-glu- | tig-the-ole- |
| **4** | glu-glu- | tig-mda-cym- |
| **5** | glu-glu-glu- | tig-cym-cym- |
| **6** | glu-glu-glu- | tig-ole-cym-cym-cym- |
| **7** | glu-glu-glu- | tig-ole-cym- |
| **8** | glu-glu-glu- | ang-mda-cym- |
| **9** | glu-glu-glu- | tig-the-cym- |
| **10** | glu-glu-glu- | tig- |
| **11** | glu-glu-glu- | tig-cym- |
| **12** | glu-glu-glu- | tig-the-ole-cym- |
| **13** | glu-glu-glu- | tig-the-cym-cym- |
| | | |
|---|---|---|
| ole = Oleandrose, cym = Cymarose, glu = Glucose, mda = 3-O-methyl-6-deoxyallose, tig = Tigloyl, ang = Angeloyl, the = Thevetose. | | |

2. Verfahren gemäß Anspruch 1, wobei vorwiegend Fettsäuren und weiteres Lipid-Material in einer ersten Extraktion mit flüssigem Kohlendioxid bei einem ersten Druck extrahiert werden, gefolgt von einer Extraktion von steroidalem Glycosid nach Formal 1 mit superkritischem Kohlendioxid bei einem zweiten Druck, während steroidale Glycoside, ausgewählt aus Verbindungen 3 bis 13 oder anderen Verbindungen, in der Pflanzenmatrix verbleiben.

3. Verfahren gemäß Anspruch 1 oder Anspruch 2, wobei das Kohlendioxid zusammen mit einer relativ kleinen Menge eines oder mehrerer wässrigen oder organischen Cosolvens verwendet wird, wobei das Cosolvens in einer Menge von weniger als etwa 10 Gew.-% des Kohlendioxids verwendet wird.

4. Verfahren gemäß Anspruch 3, wobei das Cosolvens ausgewählt wird aus Wasser, Methanol, Ethanol, Hexan, Heptan und Mischungen oder Kombinationen davon.

5. Verfahren gemäß irgendeinem der vorherigen Ansprüche, wobei die Pflanze eine Pflanze der Gattung *Hoodia* ist.

6. Verfahren gemäß Anspruch 5, wobei die Pflanze ausgewählt ist aus *Hoodia gordonii, Hoodia currorii* subsp. *currorrii* und *Hoodia currorii* subsp. *lugardii.*

7. Verfahren gemäß Anspruch 6, wobei die Pflanze *Hoodia gordonii* ist.

8. Verfahren gemäß irgendeinem der vorherigen Ansprüche, wobei ein anfänglich hergestelltes Extrakt einer oder mehreren darauf folgenden Lösungsmittelextraktionen unter Verwendung eines oder mehrerer organischer Lösungsmittel ausgesetzt ist, zum Entfernen von verbliebenen unerwünschten Verbindungen.

9. Verfahren gemäß Anspruch 8, wobei das eine oder mehrere organische Lösungsmittel ausgewählt ist aus Azeton, Ethylazetat, Heptan, Ethanol, Hexan und Mischungen oder Kombinationen davon.

10. Verfahren gemäß Anspruch 8, wobei das eine oder mehrere organische Lösungsmittel ausgewählt ist aus Azeton, Heptan und Mischungen oder Kombinationen davon.

## Revendications

1. Procédé pour séparer sélectivement un glycoside stéroïdien de formule 1 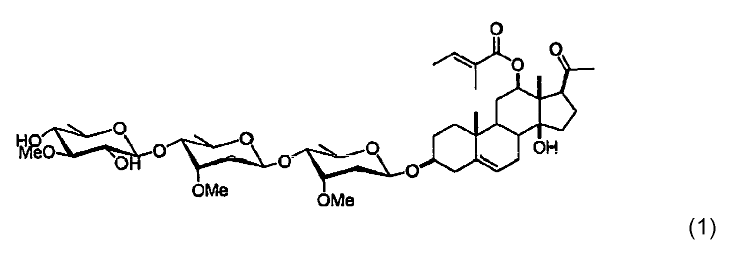 parmi un ou plusieurs glycosides stéroïdiens sélectionnés parmi les composés 3 à 13 tels que définis ci-dessous ou d'autres composés, présents dans de la matière végétale de la famille Asclepiadaceae contenant ceux-ci, le procédé comprenant la mise en contact de la matière végétale ou de matière dérivée de celle-ci, désignée ci-après par « la matière », avec du dioxyde de carbone liquide ou supercritique dans des conditions par lesquelles le glycoside stéroïdien de formule 1 se dissout dans le dioxyde de carbone liquide ou supercritique de préférence à l'un ou les composés 3 à 13 ou d'autres composés, et la récupération subséquente du glycoside stéroïdien de formule 1 de la solution de dioxyde de carbone extraite, essentiellement exempt de tout composant au goût désagréable de la matière et essentiellement exempt d'un ou plusieurs des composés 3 à 13 : 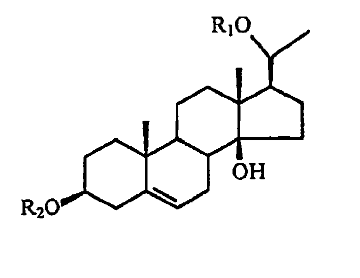
| **Composé** | **R₁** | **R₂** |
|---|---|---|
| **3** | glu-glu-glu- | tig-the-ole- |
| **4** | glu-glu- | tig-mda-cym- |
| **5** | glu-glu-glu- | tig-cym-cym- |
| **6** | glu-glu-glu- | tig-ole-cym-cym-cym- |
| **7** | glu-glu-glu- | tig-ole-cym- |
| **8** | glu-glu-glu- | ang-mda-cym- |
| **9** | glu-glu-glu- | tig-the-cym- |
| **10** | glu-glu-glu- | tig- |
| **11** | glu-glu-glu- | tig-cym- |
| **12** | glu-glu-glu- | tig-the-ole-cym- |
| **13** | glu-glu-glu- | tig-the-cym-cym- |
| | | |
|---|---|---|
| ole = oléandrose, cym = cymarose, glu = glucose, mda = 3-O-méthyl-6-désoxyallose, tig = tigloyle, ang = angéloyle, the = thévétose. | | |

2. Procédé selon la revendication 1, dans lequel des acides gras et une autre matière lipidique sont principalement extraits dans une première extraction utilisant du dioxyde de carbone liquide à une première pression, suivie d'une extraction de glycoside stéroïdien de formule 1 utilisant du dioxyde de carbone supercritique à une seconde pression tout en laissant des glycosides stéroïdiens sélectionnés parmi les composés 3 à 13 ou d'autres composés avec la matrice végétale.

3. Procédé selon la revendication 1 ou la revendication 2, dans lequel le dioxyde de carbone est utilisé en conjonction avec une quantité relativement faible d'un ou plusieurs co-solvants aqueux ou organiques, dans lequel le co-solvant est utilisé en une quantité inférieure à environ 10 % en poids du dioxyde de carbone.

4. Procédé selon la revendication 3, dans lequel le co-solvant est sélectionné parmi l'eau, le méthanol, l'éthanol, l'hexane, l'heptane et tout mélange ou toute combinaison de ceux-ci.

5. Procédé selon l'une quelconque des revendications précédentes, dans lequel la plante est une plante du genre Hoodia.

6. Procédé selon la revendication 5, dans lequel la plante est sélectionnée parmi Hoodia gordonii, Hoodia currorrii sous-espèce currorrii et Hoodia currorii sous-espèce lugardii.

7. Procédé selon la revendication 6, dans lequel la plante est Hoodia gordonii.

8. Procédé selon l'une quelconque des revendications précédentes, dans lequel un extrait initialement produit est soumis à une ou plusieurs extractions par solvant subséquentes en utilisant un ou plusieurs solvants organiques pour éliminer tout composé résiduel indésirable.

9. Procédé selon la revendication 8, dans lequel le ou les solvants organiques sont sélectionnés parmi l'acétone, l'acétate d'éthyle, l'heptane, l'éthanol, l'hexane et tout mélange ou toute combinaison de ceux-ci.

10. Procédé selon la revendication 8, dans lequel le ou les solvants organiques sont sélectionnés parmi l'acétone, l'heptane et tout mélange ou toute combinaison de ceux-ci.
